# EUROPEAN PATENT APPLICATION

(11) **EP 2 295 087 A1**
(43) Date of publication of application: **16.03.2011**
(21) Application number: 09757642.5
(22) Date of filing: 01.06.2009
(51) Int. Cl.: A61L 2/10, A47B 77/00, A47L 23/00

(54) **NOVEL ULTRAVIOLET DISINFECTION DEVICE AND/OR OZONE GENERATOR**

(30) Priority: 02.06.2008 ES 200801816
(71) Applicant: Kaparazoom, S.L.U., 45980-Paterna (VALENCIA) (ES)
(72) Inventor: SALVADOR BAREA, Francisco, E-CELLA 44370 TERUEL (ES)
(74) Representative: Isern-Jara, Nuria
(86) International application number: PCT/ES2009/000305
(87) International publication number: WO 2009/147263

(57) **Abstract**

The invention relates to a novel ultraviolet disinfection device and/or ozone generator which can be used to improve hygiene in relation to different pieces of equipment, using at least one ultraviolet light and/or an ozone generator in order to irradiate said equipment. The invention includes versions in which the source of radiation emanates from inside traditional holders, preferably made from a light-conducting plastic material, thereby enabling objects that have been washed in a traditional manner to remain aseptic until use. The invention also includes versions in which a traditional lamp holder, supporting element or strip is used to incorporate the light source into drawers or cupboards, for the hygienisation of the objects contained therein, as well as versions of the radiation device for disinfecting footwear in shoe racks. The invention is suitable for the hygienisation of household goods and equipment in general and hospital equipment.

## Description

### Object of the invention:

The new ultraviolet and/or ozone-generating disinfection device presents the novelty of using at least one ultraviolet light and/or ozone-generating source that irradiates from the inside or outside of conventional supports used for holding or storing houseware or other utensils, these being of a preferably plastic luminoconductive nature and therefore permeable to said radiation. As in the cutlery tray or dispenser (figures 1 and 3), the cutlery draining baskets (figure 4) or with footwear by use of the support of (figure 14), it offers the user greater sterilization safety because it takes advantage of the germicide action of ultraviolet radiation and/or ozone generation. Versions are contemplated wherein ultraviolet radiation and/or ozone generation are incorporated by use of a conventional support in chest drawers (figure 6), cupboards (figure 10) and shoe chests (figure 13).

The invention relates to the industrial sector of household goods and to the hospital sector.

### Background of the invention:

Nowadays, household utensils are hygienized by using conventional cleaning procedures with detergents. After these procedures, the material is left in different supports or containers until used. In the case of the application to footware, the new device for disinfection by ultraviolet and/or ozone-generating light improves hygiene, also preventing the proliferation of germs and bad odour-causing bacteria.

There are no known teachings in the state of the art of the incorporation of at least one ultraviolet light and/or ozone-generating source irradiating in said type of supports with the aim of maintaining asepsis of household utensils. In the same way, the use of ultraviolet light and/or ozone generation for the inside of cupboards and chest drawers is not known. Therefore, it would be desirable to obtain a hygienic guarantee for houseware from the time they are washed to when they are used, also for footware and clothes, given that storage in today's supports intended to this end does not guarantee that gear placed in them is not contaminated by pathogen agents during said time period. This is specially relevant for clinical environments. To this end, the present invention offers a guarantee of asepsis by irradiating said gear with at least one ultraviolet light and/or ozone-generating source, given the very high number of microorganisms that can be killed by using this radiation.

The integration of ultraviolet radiation and/or ozone-generation in conventional supports for houseware of the present invention allows to obtain a new final product satisfying the conditions required to guarantee the correct asepsis of houseware elements.

### Description of the invention:

The new ultraviolet and/or ozone-generating disinfection device comprises at least one ultraviolet radiation and/or ozone-generating source (Ia) irradiating from the inside of conventional supports (figures 1 and 2). Another disinfection device uses ultraviolet radiation and/or ozone-generation (1b), irradiated from the outside of conventional supports (figure 3). Another version of the ultraviolet radiation and/or ozone-generating device (1c) irradiates from the inside of conventional supports (figures 4 and 5).

These conventional supports for holding or storing houseware or other utensils are preferably of plastic nature with light-conductive properties, allowing the light source to irradiate uniformly the gear held in them; as well as footware by use of the support (12), which is also of light-conductive plastic nature and hence permeable to said ultraviolet radiation and/or ozone-generation (figure 14).

In other applications it will suffice that the light radiation (Id) is confined to an opaque assembly, such as in a chest of drawers (3) (figure 6); to this end, the incorporation of at least one ultraviolet light and/or ozone-generating source (Id) is contemplated, irradiating from a conventional strip (figures 7 and 8) located near the gear to be sterilized (5) and in cupboards (figures 9 and 10) in which the light source (Ie) irradiates the gear (5) from a conventional support (6).

The light radiation source is fed by batteries (7) (figure 2) or directly from the power grid (4) (figure 4). The activation periods of said radiation can be automated if necessary, using a conventional 24-hour programmer.

In addition, with an aim to protecting the user from this potentially damaging light emission, different safety systems or devices (11) are used that block this radiation when the door of the cupboard or chest of drawers in which the light radiation source is installed is in an open state.

### Description of the drawings

As a complement of the description being made and for a better understanding of the characteristics of the invention, attached to the present descriptive memory and as an integral part of it is a set of drawings where, for purposes of illustration and in a non-limiting manner, the following is shown:
Figure 1 shows the cutlery tray (2) before being placed in a conventional drawer (3), in which (8) are the cavities where the gear is housed, (10) represents a switch and (11), a safety element.
Figure 2 shows a section of the cutlery-holding tray (2), in which the source of light radiation (Ia) and the power source (7) can be seen in the inside; (10) represents a switch and (11), a safety element.
Figure 3 shows the cutlery tray (2), in which the source of light radiation external to the tray (1 b) can be seen. in which (8) are the cavities where the gear is housed, (10) represents a switch and (11), a safety element.
Figure 4 shows a cutlery-draining support (5), fed to the electrical power grid (4) with its switch (10).
In figure 5, the source of light radiation (1c) in the inside can be seen.
Figure 6 shows a section of a conventional drawer (3), a household utensil (5) and the sources of light radiation (Id).
In figures 7 and 8, a detail of the assembly that houses the source of light radiation (10) (Id) is shown.
In figure 9 a cupboard is shown, and figure 10 represents the inside thereof, in which the houseware (5) is shown, as well as the support from where ultraviolet and/or ozone-generating light (6) is irradiated.
Figure 11 shows the support (6), in which the source of light radiation (Ie) is housed.
In figure 12 a front view is shown of a shoe cabinet with the door open (9) and supports for difusing ultraviolet and/or ozone-generating light (12).
Figure 13 shows a section of the shoe cabinet with the door closed (9) and footware placed on the support for difusing ultraviolet and/or ozone-generating light (12).
Figure 14 shows a section of the diffusor support for footware (12), containing 25 the emission source of ultraviolet and/or ozone-generating (19).

### Preferred embodiment of the invention

The ultraviolet and/or ozone-generation disinfection device for houseware described in the following preferred embodiment (figures 1 and 2) comprises at least one ultraviolet light and/or ozone-generating source (1 a) in the inside of a body of plastic light-conductive nature (2), i.e. permeable to said radiation, provided with a battery power source (7) housed in the plastic tray (2). The previously-washed gear is placed in the cavities (8) in the support (2). By activating the source of light radiation, the radiation is emitted from the ultraviolet and/or ozone-generating source (Ia), to the walls or surfaces of the tray, distributing the light radiation in an homogeneous manner over the surfaces of the support, which is preferably of a light-conductive plastic material.

The activation of the ultraviolet and/or ozone-generation disinfection device for houseware is performed by use of a conventional switch (10), and the conventional end-of-run (11), turning on the light source only in the case that the drawer is closed, thus preventing radiation exposure to the user.

The materials, shape, size and arrangement of the elements may be modified as long as this does not imply altering the essential characteristics of the claimed invention.

## Claims

1. Disinfection device of the type that uses ultraviolet radiation and/or ozone-generating type for killing microorganisms and pathogen agents, **characterized in that** it comprises at least one ultraviolet light and/or ozone-generating source (Ia), which irradiates from the inside of a gear-containing support. The material of the supports preferably being of light-conductive plastic nature and permeable to radiation.

2. Disinfection device of the type that uses ultraviolet radiation and/or ozone-generating type for killing microorganisms and pathogen agents, **characterized in that** it comprises at least one ultraviolet light and/or ozone-generating source (1b), which irradiates from the outside of the material making up the conventional houseware tray (2) to the cavities for houseware (8) in which the gear to be sterilized is placed.

3. Disinfection device of the type that uses ultraviolet radiation and/or ozone-generating type for killing microorganisms and pathogen agents, **characterized in that** it comprises at least one ultraviolet light and/or ozone-generating source (1c), which irradiates the inside of a houseware-containing support of the type used for draining household cutlery. The material of the supports preferably being of a light-conducting plastic permeable to radiation.

4. Disinfection device of the type that use ultraviolet radiation and/or ozone-generating type for killing microorganisms and pathogen agents, **characterized in that** it comprises at least one ultraviolet light and/or ozone-generating source (1 d) that irradiates from a conventional strip located in close proximity to the gear to be sterilized (5), housed in a conventional drawer for houseware (3).

5. Disinfection device of the type that uses ultraviolet radiation and/or ozone-generating type for killing microorganisms and pathogen agents, **characterized in that** it comprises at least one ultraviolet light and/or ozone-generating source (1 e) irradiating from a conventional strip located in close proximity to the gear to be sterilized (5) housed in a conventional cupboard.

6. Disinfection device of the type that uses ultraviolet radiation and/or ozone-generating type for killing microorganisms and pathogen agents, **characterized in that** it comprises at least one ultraviolet light and/or ozone-generating source, said light radiation being emitted in the inside of footware, being located on a support (12), which additionally, by virtue of its geometry, fulfills the function of support for the footware, said support preferably being made up of a light-conductive plastic material.
